# EUROPÄISCHE PATENTSCHRIFT

(11) **EP 2 370 397 B1**
(45) Veröffentlichungstag und Bekanntmachung des Hinweises auf die Patenterteilung: **16.01.2013**
(21) Anmeldenummer: 09748809.2
(22) Anmeldetag: 12.11.2009
(51) Int. Cl.: C07C 231/24, C07D 201/16, C07D 207/267

(54) **VERFAHREN ZUR ENTFERNUNG VON NEBENPRODUKTEN AUS N-VINYLAMIDEN**
PROCESS FOR REMOVING BY-PRODUCTS FROM N-VINYLAMIDES
PROCÉDÉ POUR ÉLIMINER DES PRODUITS SECONDAIRES DANS DES N-VINYLAMIDES

(30) Priorität: 28.11.2008 EP 08170175
(43) Veröffentlichungstag der Anmeldung: 05.10.2011
(73) Patentinhaber: BASF SE, 67056 Ludwigshafen (DE)
(72) Erfinder: STAFFEL, Wolfgang, 67165 Waldsee (DE); VOGELSANG, Regina, 67063 Ludwigshafen (DE); KESSINGER, Roland, 69469 Weinheim (DE); TUTTELBERG, Lembit, 68305 Mannheim (DE); HEIDA, Bernd, 67158 Ellerstadt (DE)
(86) Internationale Anmeldenummer: PCT/EP2009/065038
(87) Internationale Veröffentlichungsnummer: WO 2010/060801

(56) Entgegenhaltungen:
- WO-A2-01/90066
- DE-A1- 3 938 016
- JP-A- 61 065 853
- JP-A- 61 289 069

## Beschreibung

Die Erfindung betrifft ein Verfahren zur Abtrennung von Nebenprodukten aus N-Vinylamid reichen Produktgemischen (Roh-N-Vinylamid), welches dadurch gekennzeichnet ist, dass eine Extraktion des Roh-N-Vinylamids mit einem organischen Lösemittel als Extraktionsmittel durchgeführt wird.

Aus DE 39 38 016 A1, JP 61 289069 A und JP 61 065853 sind Verfahren zur Reinigung von N-Vinylverbindungen durch Extraktion mit Wasser bekannt.

Herstellverfahren für N-Vinylamide, z.B. auch N-Vinylpyrrolidon, sind z.B. in DE-A 102 55 437, DE-A 195 09 362 und DE-A 198 39 565 beschrieben. Bedingt durch die Herstellverfahren enthält das erhaltene Produktgemisch eine Reihe unterschiedlicher Nebenprodukte, z.B. Ausgangsstoffe, bereits gebildete Oligomere oder Polymere der Vinylverbindungen und sonstige Nebenprodukte, die insbesondere bei der Durchführung der chemischen Umsetzung im Reaktor entstehen können. Zu letzteren gehören auch fluoreszierende Nebenprodukte, die bei einer üblichen destillativen Aufarbeitung des Produktgemisches nicht oder nur unvollständig entfernt werden.

Diese fluoreszierenden Nebenprodukte vermindern die Produktqualität. Gewünscht ist daher ein einfaches Verfahren, diese fluoreszierenden Nebenprodukte möglichst vollständig aus dem Produktgemisch zu entfernen.

Demgemäß wurde das eingangs definierte Verfahren gefunden.

Bei dem erfindungsgemäßen Verfahren werden Nebenprodukte aus N-Vinylamid reichen Produktgemischen (Roh-N-Vinylamid) durch Extraktion abgetrennt.

### Zum Roh-N-Vinylamid

Bei dem N-Vinylamid kann es sich um cyclische N-Vinylamide (Vinyllactame) oder um nicht cyclische N-Vinylamide, z.B. solche der Formel 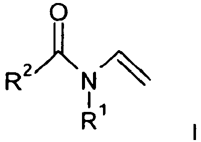 handeln.

In Formel I stehen R1 und R2 unabhängig voneinander für ein H Atom oder eine C1 bis C10, vorzugsweise für einen C1 bis C4 Alkylgruppe.

Als nicht cyclische N-Vinylamide der Formel I seien insbesondere N-Vinylformamid (R1 und R2 = H) und N-Vinyl-N-methylacetamid (VIMA, R1 und R2 = Methyl) genannt.

Als Vinyllactame seien insbesondere N-Vinylpiperidon, N-Vinylcaprolactam, N-Vinyl-pyrrolidon oder deren Derivate genannt. Bei den Derivaten handelt es sich insbesondere um Vinyllactame, bei denen ein oder mehrere, vorzugsweise ein oder zwei Kohlenstoffatome des Ringsystems durch C1 bis C10 Alkylgruppen substituiert sind, genannt seien insbesondere N-Vinylmethylpyrrolidon, N-Vinyldimethylpyrrolidon oder N-Vinylethylpyrrolidon.

Besonders bevorzugte N-Vinylamide sind Vinyllactame, insbesondere N-Vinylcaprolactam oder N-Vinylpyrrolidon.

Ganz besonders bevorzugtes N-Vinylamid ist N-Vinylpyrrolidon.

Erfindungsgemäß wird ein Roh-N-Vinylamid extrahiert. Unter Roh-N-Vinylamid wird ein N-Vinylamid reiches Produktgemisch verstanden. Es kann sich dabei um das Produktgemisch handeln, welches als Produkt der chemischen Umsetzung im Reaktor entsteht, anschließend als Reaktoraustrag entnommen wird und unmittelbar der Extraktion zugeführt wird. Im Falle des N-Vinylpyrrolidon ist dies vorzugsweise das Produktgemisch, welches bei der Umsetzung von 2- Pyrrolidon (gamma-Butyrolactam) mit Acetylen erhalten wird.

Es kann sich aber auch um Roh-N-Vinylamid aus anderen Herstellverfahren handeln, wie sie z.B. in WO 2006/109869 auf Seite 5 beschrieben sind. Roh-N-Vinylamid ist z.B. durch Umsetzung von Butyrolacton mit Ethanolamin, Substitution der Hydroxygruppe durch ein Chlorid mit Thionylchlorid und anschließende Dehydratation oder durch Umsetzung von N-(2-Hydroxyethyl)-2-pyrrolidon mit Essigsäureanhydrid und Abspaltung von Essigsäure oder durch Gasphasendehydration von N-Hydroxyethyl-2-pyrrolidon erhältlich.

Bei dem für die Extraktion verwendeten Roh-N-Vinylamid kann es sich auch um ein Produktgemisch handeln, welches nach der Umsetzung im Reaktor bereits weiter aufgearbeitet wurde, um Ausgangsstoffe oder Nebenprodukte zu entfernen.

Insbesondere handelt es sich bei dem Roh-N-Vinylamid, z.B. dem Roh-N-Vinyl-pyrrolidon, um ein Gemisch, welches durch Umsetzung von Ausgangsstoffen, z.B. 2-Pyrrolidon, mit Acetylen und gegebenenfalls eine anschließende Aufarbeitung des erhaltenen Produktgemisches erhalten wurde.

Generell besteht das als Roh-N-Vinylamid für die Extraktion verwendete Gemisch zu mindestens 50 Gew.-%, besonders bevorzugt zu mindestens 70 Gew.-% und insbesondere zu mindestens 80 Gew.-% aus dem N-Vinylamid.

Im Falle des N-Vinylpyrrolidon wird besonders bevorzugt ein Roh-N-Vinylpyrrolidon verwendet, welches zunächst durch Umsetzung von Acetylen mit 2-Pyrrolidon erhalten wurde und aus dem anschließend Schwersieder (z.B. Oligomere des N-Vinyl-pyrrolidon) durch eine erste Destillation (Rohdestillation) abgetrennt wurden. Das abdestillierte Gemisch, enthält N-Vinylpyrrolidon, im Allgemeinen in Mengen größer 70 Gew.-%, besonders bevorzugt größer 80 Gew.-%, sowie Restmengen an 2-Pyrrolidon (im Allgemeinen 0 bis 30 Gew.-%, besonders bevorzugt 1 bis 25 Gew.-%) und Nebenprodukte mit einem Siedepunkt oberhalb des 2-Pyrrolidon, im Allgemeinen in Mengen von 0,5 bis 20 Gew.-%, besonders bevorzugt 0,5 bis 10 Gew.-%, wobei alle vorstehenden Gewichtsangaben auf das Gemisch bezogen sind. Dieses Gemisch wird in einer besonders bevorzugten Ausführungsform als Roh-N-Vinylpyrrolidon für die anschließende Extraktion verwendet.

Das für die Extraktion verwendete Roh-N-Vinylamid, z.B. Roh-N-Vinylpyrroplidon, enthält insbesondere fluoreszierende Nebenprodukte. Bei diesen Nebenprodukten handelt es sich möglicherweise um aromatische Verbindungen mit einem Siedepunkt zwischen 150 und 320°C, insbesondere 200 bis 260°C (bei Normaldruck).

### Zum Extraktionsmittel

Bei der Extraktion wird ein organisches Lösemittel als Extraktionsmittel verwendet.

Die abzutrennenden Nebenprodukte sollen in dem Lösemittel möglichst gut löslich sein. Gleichzeitig darf das Lösemittel mit dem zu extrahierenden Gemisch unter den Extraktionsbedingungen nicht oder nur wenig mischbar sein, so dass sich zwei Phasen ausbilden. Das Phasendiagramm des binären Systems aus zu extrahierendem Gemisch und Extraktionsmittel muss unter den Extraktionsbedingungen eine Mischungslücke aufweisen.

Falls das Roh-N-Vinylamid mit dem Extraktionsmittel keine Mischungslücke aufweist, kann statt des Roh-N-Vinylamids ein Gemisch verwendet werden, welches eine entsprechende Mischungslücke aufweist. Die Mischungskomponente sollte mit dem Roh-N-Vinylamid möglichst gut mischbar, mit dem Extraktionsmittel möglichst schlecht mischbar und nach der Extraktion aus dem Roh-N-Vinylamid leicht abtrennbar sein. Als Mischungskomponente bevorzugt ist Wasser. Es kann sich dabei auch um Wasser handeln, welches anorganische oder organische Salze in gelöster Form enthält.

In einer bevorzugten Ausführungsform der Erfindung erfolgt die Extraktion unter Zusatz von Wasser. Das Extraktionsmittel besteht dann aus Wasser und einem oder mehreren organischen Lösemitteln. Das Extraktionsmittel kann z. B. zu 1 bis 70 Gew. %, insbesondere 2 bis 50 Gew. % aus Wasser bestehen.

Als Extraktionsmittel geeignet sind z. B. aromatische Lösemittel wie Benzol, Toluol oder Xylol (Isomerengemisch). Aromatische Lösemittel sind jedoch häufig unerwünscht. Weiterhin ist im vorliegenden Fall auch nachteilig, dass N-Vinylpyrrolidon teilweise in die organische Phase übergeht und daher keine vollständige Abtrennung des N-Vinylpyrrolidon erfolgt.

Als organische Lösemittel für das Extraktionsmittel sind insbesondere aliphatische oder cycloaliphatische Lösemittel geeignet. Besonders bevorzugt sind bei 21°C (1 bar) flüssige aliphatische oder cycloaliphatische Lösemittel mit einem Siedepunkt größer 80°C (bei 1 bar). Insbesondere handelt es sich um völlig unpolare Lösemittel, d.h. um Verbindungen, die nur aus Kohlenstoff und Wasserstoff bestehen (Kohlenwasserstoffe).

Die vorstehenden aliphatischen oder cycloaliphatischen Lösemittel können natürlich auch im Gemisch mit aromatischen Lösemitteln verwendet werden. Besonders bevorzugt ist der Gehalt an aromatischen Lösemitteln in der Gesamtmenge aller im Extraktionsmittel verwendeten organischen Lösemittel kleiner 50 Gew. %, insbesondere kleiner 30 Gew. %, besonders bevorzugt kleiner 10 Gew. %, beziehungsweise kleiner 5 Gew. %. In einer besonders bevorzugten Ausführungsform enthalten die als Extraktionsmittel verwendeten organischen Lösemittel keine aromatischen Lösemittel.

Als geeignete aliphatische oder cycloaliphatische Lösemittel genannt seien z.B. Methylcyclopentan, Cyclopentan, Hexan, Methylcyclohexan, Cyclohexan, Heptan oder Octan.. Als Lösungsmittel eignen sich insbesondere Verbindungen, die mit dem zu extrahierenden N-Vinylamid eine Mischungslücke aufweisen. Es können auch Gemische unterschiedlicher Lösemittel als Extraktionsmittel verwendet werden; genannt seien z. B. Gemische von Cyclohexan und Methylcyclohexan in beliebigen Verhältnissen.

Als besonders bevorzugtes organisches Lösemittel seien Methylcyclohexan, Cyclohexan, Methylcyclopentan und Cyclopentan und deren Gemische genannt. Ganz besonders bevorzugt sind Gemische aus Metylcyclopentan und Cyclopentan oder Gemische aus Methylcyclohexan und Cyclhexan.

Ganz besonders bevorzugt sind Extraktionsmittel, welche Wasser und die vorstehenden besonders bevorzugten organischen Lösemittel enthalten oder daraus bestehen. In einer besonderen Ausführungsform wird ein Extraktionsmittel, welches Methylcyclohexan und Wasser enthält oder daraus besteht, als Extraktionsmittel für Roh-N-Vinylpyrrolidon verwendet.

Ein Gemisch aus Roh-N-Vinylpyrrolidon und Wasser hat mit Methylcyclohexan unter geeigneten Extraktionsbedingungen eine Mischungslücke. Mischungslücken in ternären Systemen lassen sich in üblicher Weise Variation der Konzentration ermitteln und in Dreiecksdiagrammen darstellen

### Zur Durchführung der Extraktion

Generell ist für die Extraktion auch der Roh-N-Vinylamide ein Temperaturbereich von 20 bis 100°C unter Normaldruck (1 bar) geeignet. Die Extraktion kann auch bei erhöhtem Druck oder Unterdruck durchgeführt werden. Das Extraktionsmittel kann z. B. in einer Menge von 20 bis 0,5 Gewichtsteilen, insbesondere in einer Menge von 10 bis 1 Gewichtsteilen, auf 1 Gewichtsteil Roh-N-Vinylpyrrolidon verwendet werden.

Geeignete Extraktionsbedingungen ergeben sich bei dem ternären System aus Roh-N-Vinylpyrrolidon, Wasser und Methylcyclohexan innerhalb eines Temperaturbereichs von 20 bis 100°C, besonders bevorzugt 30 bis 80°C bei 1 bar und einem Gewichtsanteil von
10 bis 50 Gew.-%, besonders bevorzugt 20 bis 40 Gew.-% Roh-N-Vinylpyrrolidon,
20 bis 80 Gew.-%, besonders bevorzugt 30 bis 70 Gew.-% Methylcyclohexan und
2 bis 50 Gew.-%, besonders bevorzugt 10 bis 30 Gew.-% Wasser.

Entsprechend wird die Extraktion mit dem ternären System aus Roh-N-Vinylpyrrolidon, Wasser und Methylcyclohexan vorzugsweise unter Einhaltung der vorstehenden Extraktionsbedingungen durchgeführt.

Die Extraktion kann diskontinuierlich oder kontinuierlich durchgeführt werden.

Bei diskontinuierlicher Extraktion verwendet man im einfachen Fall einen Rührbehälter zum Mischen der beiden Phasen (Extraktionsmittel und zu extrahierende Phase) und einen Abscheider, in dem sich die beiden Phasen entsprechend ihrer Dichte in eine obere und untere Phase auftrennen (Mixer-Settler). Das Lösemittel kann danach gereinigt werden, z.B. durch Destillation, und erneut in den Mixer zurückgeführt werden.

Die Extraktion wird vorzugsweise kontinuierlich durchgeführt. Insbesondere wird das gesamte Herstellungsverfahren, umfassend die Umsetzung der Ausgangsstoffe im Reaktor und anschließende Aufarbeitung, kontinuierlich durchgeführt.

Zur kontinuierlichen Durchführung der Extraktion eignen sich ebenso Mixer-Settler wie vorstehend beschrieben oder Kaskaden von Mixer-Settlern; in einer bevorzugten Ausführungsform verwendet man bei kontinuierlicher Durchführung Kolonnen, z.B. übliche Extraktionskolonnen, wie Sprühbodenkolonnen, Siebbodenkolonnen, Füllkörper-Extraktionskolonnen, Pulsationskolonnen oder Rotationskolonnen. Die Ausgangskomponenten werden über sogenannte Verteiler zugeführt. Verteiler sind Einbauten in der Kolonne, welche eine möglichst gute Verteilung der Komponenten über den Kolonnenquerschnitt bewirken. Vorzugsweise sind geeignete Verteiler horizontal in die Kolonnen eingebaut.

Die kontinuierliche Extraktion kann in einer oder mehreren Kolonnen durchgeführt werden. Für das ternäre System Roh-N-Vinylpyrrolidon / Wasser / Methylcyclohexan kommt z.B. eine Durchführung der Extraktion in einer Extraktionskolonne und einer sich daran anschließenden Waschkolonne in Betracht. In der Extraktionskolonne werden die unerwünschten Nebenprodukte mit Methylcyclohexan aus dem Wasser/Roh-N-Vinylpyrrolidon - Gemisch abgetrennt; in der Waschkolonne N-Vinylpyrrolidon, welches (unerwünschter Weise) ebenfalls in die Methylcyclohexan-Phase übergegangen ist, wieder mit Wasser zurückgewonnen. Das in der Waschkolonne abgetrennte Wasser/N-Vinylpyrrolidon Gemisch kann in die Extraktionskolonne zurückgeführt werden.

In einer bevorzugten Ausführungsform wird die kontinuierliche Extraktion in einer Kolonne durchgeführt.

Die Zufuhr des Roh-NVP, des Extraktionsmittels und gegebenenfalls des Wassers kann an beliebigen Stellen der Kolonne bzw. der Kolonnen erfolgen. Roh-NVP, Extraktionsmittel und ggf. Wasser können getrennt oder gemeinsam zugeführt werden, es können alle drei Komponenten oder zwei der drei Komponenten zusammen an beliebigen Stellen der Kolonnen zugeführt werden.

Vorzugsweise wird das Extraktionsmittel (Methylcyclohexan) über einen Verteiler im unteren Teil der Kolonne und Wasser über einen Verteiler im oberen Teil der Kolonne zugeführt, während das zu extrahierende Roh-N-Vinylpyrrolidon im mittleren Teil zugeführt wird. Entsprechend der Dichte der Komponenten steigt das Extraktionsmittel Methylcyclohexan zum Kolonnenkopf und reichert sich mit den aus dem Roh-N-Vinyl-pyrrolidon zu extrahierenden Nebenprodukten und auch mit N-Vinylpyrrolidon an; im oberen Teil der Kolonne wird N-Vinylpyrrolidon mit Wasser aus dem Methylcyclohexan ausgewaschen, so dass am Kopf ein weitgehend N-Vinylpyrrolidon freies Gemisch von Methylcyclohexan und extrahierten Nebenprodukten abgezogen werden kann. Am Sumpf der Kolonne wird ein Gemisch aus gereinigtem Roh-N-Vinylpyrrolidon und Wasser abgezogen.

Das sich dabei in der Kolonne einstellende Temperaturprofil hängt von den Mischungsenthalpien der Komponenten und der Verteilung der Komponenten in der Kolonne ab. Bei dem ternären System Roh-N-Vinylpyrrolidon / Wasser / Methylcyclohexan beträgt die Temperatur in der Kolonne vorzugsweise 10 und 90 °C, insbesondere 20 bis 60 °C und besonders bevorzug 30 bis 50°C.

Methylcyclohexan kann anschließend durch Destillation gereinigt und wieder verwendet werden.

Auch das Wasser/ Roh-N-Vinylpyrrolidon- Gemisch kann weiter aufgearbeitet werden.

Das durch Extraktion gereinigte Roh-N-Vinylamid, insbesondere Roh-N-Vinylpyrrolidon enthält kaum noch fluoreszierende Nebenprodukte. Nach Abtrennung des Wassers ist der Gehalt kleiner 0,5 Gew.-Teile, insbesondere kleiner 0,1 Gew.-Teile und besonders bevorzugt kleiner 0,05 Gew.-Teile bzw. kleiner 0,01 Gew.-Teile, bezogen auf 100 Gew.-Teile N-Vinylpyrrolydon.

### Beispiele

N-Vinylpyrrolidon wurde durch Umsetzung von Acetylen mit 2-Pyrrolidon hergestellt. Aus dem erhaltenen Gemisch wurden Schwersieder durch Destillation abgetrennt. Als Kopfprodukt wird ein N-Vinylpyrrolidon erhalten, welches noch fluoreszierende Verunreinigungen enthält. Dieses wurde als Roh-N-Vinylpyrrolidon bei den nachfolgenden Extraktionsversuchen eingesetzt.

200 Milliliter Roh-N-Vinyl-pyrrolidon (Roh-NVP), 200 Milliliter Wasser and 200 Milliliter jeweils eines der in Tabelle 1 aufgeführten Lösemittel als Extraktionsmittel wurden intensiv gemischt und geschüttelt. Es wurde eine wässrige und eine organische Phase erhalten. Die Phasen wurden getrennt und die Fluoreszens der wässrigen Phase, welche das N-Vinylpyrrolidon enthält (NVP-extrahiert), bestimmt.

Die ermittelte Fluorszens (F) des Roh-N-Vinylpyrrolidon wurde zur Fluoreszens der erhaltenen wässrigen Phase ins Verhältnis gesetzt:
F = F Roh-NVP/ F NVP-extrahiert.

Je größer der Wert von F, desto besser ist die erreichte Abtrennung der fluoreszierenden Verunreuinigungen. Der Wert für F ist in Tabelle 1 aufgeführt.

Die Messungen wurden mit einem Perkin Elmer Lumineszenzspektralphotometer LS50-B durchgeführt. Die zu untersuchende Substanz wurde in eine UV-Küvette (d = 1.0 cm) gefüllt und bei drei verschiedenen Anregungswellenlängen die Wellenlänge bestimmt, bei der die maximale Fluoreszens erfolgt. Die Intensität dieser maximalen Fluoreszens wurde zur Intensität der maximalen Fluoreszens des Roh-NVP ins Verhältnis gesetzt.
Temperatur: 20°C Anregungswellenlängen λex = 320 nm, 340 nm und 360 nm
Spaltweite Anregungswellenlänge/ Emissionswellenlänge: 5 nm / 5 nm

**Tabelle 1: Fluoreszens-daten**

| | | Intensität des Emissions-maximums bei | | Intensität des Emissions-maximums bei | | Intensität des Emissions-maximums bei | | |
|---|---|---|---|---|---|---|---|---|
| | | λex = 320 nm | | λex = 340 nm | | λex = 360 nm | | |
| | Extraktions-Lösemittel | absolut | F | absolut | F | absolut | F | Kommentar |
| 1 | kein | 3470* | 1 | 5208* | 1 | 3558* | 1 | |
| 2 | Benzol | 246 | 14,1 | 341 | 15,3 | 168 | 21,2 | Teil des NVP blieb in der organischen Phase |
| 3 | Toluol | 211 | 16,4 | 278,2 | 18,7 | 133,6 | 26,6 | Teil des NVP blieb in der organischen Phase |
| 4 | Xylol (Isomerengemisch) | 269 | 12,9 | 258,8 | 20,1 | 123,5 | 28,8 | Teil des NVP blieb in der organischen Phase |
| 5 | Hexan | 770 | 4,5 | >1000 | <5 | >1000 | <5 | |
| 6 | Heptan | 749,2 | 4,6 | 587 | 8,9 | 622,2 | 5,7 | |
| 7 | Octan | 753 | 4,6 | 553 | 9,4 | 566 | 6,3 | |
| 8 | Petroleumether | 357 | 9,7 | 680 | 7,7 | 419 | 8,5 | |
| 9 | Methylcyclopentan | 722 | 4,8 | 392 | 13,3 | 351 | 10,1 | |
| 10 | Methylcyclohexan | 690 | 5,0 | 387 | 13,5 | 360 | 9,9 | |
| 11 | Cyclohexan | 750 | 4,6 | 410 | 12,7 | 379 | 9,4 | |
| 12 | Diethylether | 714 | 4,9 | 950 | 5,5 | >1000 | <5 | |
| 13 | tert-Butylmethylether | 801 | 4,3 | 916 | 5,7 | 975 | 3,7 | |

| | | | | | | | | |
|---|---|---|---|---|---|---|---|---|
| * 1 Gewichtsteil Roh-NVP wurde für die Fluoreszensmessung mit 20 Gew.-Teilen Wasser verdünnt. | | | | | | | | |

## Patentansprüche

1. Verfahren zur Abtrennung von Nebenprodukten aus N-Vinylamid reichen Produktgemischen (Roh-N-Vinylamid), **dadurch gekennzeichnet, dass** eine Extraktion des Roh-N-Vinylamids mit einem organischen Lösemittel als Extraktionsmittel durchgeführt wird.

2. Verfahren gemäß Anspruch 1, **dadurch gekennzeichnet, dass** es sich bei dem N-Vinylamid um cyclische N-Vinylamide (Vinyllactame) oder um nicht cyclische N-Vinylamide der Formel 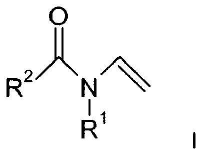 handelt, worin R1 und R2 unabhängig voneinander für ein H Atom oder eine C1 bis C10 Alkylgruppe.

3. Verfahren gemäß Anspruch 1 oder 2, **dadurch gekennzeichnet, dass** es sich bei den nicht cyclischen N-Vinylamiden der Formel I um N-Vinylformamid (R1 und R2 = H) oder N-Vinyl-N-methylacetamid (VIMA, R1 und R2 = Methyl) handelt.

4. Verfahren gemäß einem der Ansprüche 1 oder 2, **dadurch gekennzeichnet, dass** es sich bei den cyclischen N-Vinylamiden um N-Vinylpiperidon, N-Vinylcaprolactam oder N-Vinylpyrrolidon oder deren Derivate, z.B. N-Vinylmethylpyrrolidon, N-Vinyldimethylpyrrolidon oder N-Vinylethylpyrrolidon handelt.

5. Verfahren gemäß einem der Ansprüche 1 bis 4, **dadurch gekennzeichnet, dass** es sich bei dem N-Vinylamid um N-Vinylpyrrolidon handelt.

6. Verfahren gemäß einem der Ansprüche 1 bis 5, **dadurch gekennzeichnet, dass** das Roh-N-Vinylamid zu mindestens 50 Gew.-% aus dem N-Vinylamid besteht.

7. Verfahren gemäß einem der Ansprüche 1 bis 6, **dadurch gekennzeichnet, dass** das Roh-N-Vinylamid durch Umsetzung von Ausgangsstoffen mit Acetylen und gegebenenfalls eine anschließende Aufarbeitung erhalten wurde.

8. Verfahren gemäß einem der Ansprüche 1 bis 7, **dadurch gekennzeichnet, dass** es sich bei dem Roh-N-Vinylamid um Roh-N-Vinylpyrrolidon handelt, welches durch Umsetzung von 2- Pyrrolidon (gamma-Butyrolactam) mit Acetylen und gegebenenfalls eine anschließende Aufarbeitung erhalten wurde.

9. Verfahren gemäß einem der Ansprüche 1 bis 8, **dadurch gekennzeichnet, dass** vor der Extraktion eine Destillation zur Abtrennung der Schwersieder aus dem Roh-N-Vinylamid (Rohdestillation) durchgeführt wird.

10. Verfahren gemäß einem der Ansprüche 1 bis 9, **dadurch gekennzeichnet, dass** ein aliphatisches oder cycloaliphatisches Lösemittel verwendet wird.

11. Verfahren gemäß einem der Ansprüche 1 bis 10, **dadurch gekennzeichnet, dass** die Extraktion unter Zusatz von Wasser erfolgt.

12. Verfahren gemäß einem der Ansprüche 1 bis 11, **dadurch gekennzeichnet, dass** Roh-N-Vinylpyrrolidon mit Methylcyclohexan unter Zusatz von Wasser extrahiert wird.

13. Verfahren gemäß einem der Ansprüche 1 bis 12, **dadurch gekennzeichnet, dass** die Extraktion in einem Temperaturbereich von 20 bis 100°C durchgeführt wird.

14. Verfahren gemäß einem der Ansprüche 1 bis 13, **dadurch gekennzeichnet, dass** die Extraktion kontinuierlich in einer oder mehreren Kolonnen durchgeführt wird.

15. Verfahren gemäß einem der Ansprüche 1 bis 14, **dadurch gekennzeichnet, dass** Roh-N-Vinylpyrrolidon mit Methylcyclohexan unter Zusatz von Wasser kontinuierlich in einer Kolonne extrahiert wird, wobei Wasser über einen Verteiler im oberen Teil der Kolonne, Methylcyclohexan über einen Verteiler im unteren Teil der Kolonne und das Roh-N-Vinylpyrrolidon im mittleren Teil der Kolonne zugefahren wird.

16. Verfahren gemäß Anspruch 15, **dadurch gekennzeichnet, dass** am Kopf der Kolonne ein Gemisch aus Methylcyclohexan und abgetrennten Nebenprodukten und am Boden der Kolonne ein Gemisch aus gereinigtem Roh-N-Vinylpyrrolidon und Wasser abgenommen wird.

17. Verfahren gemäß einem der Ansprüche 1 bis 16, **dadurch gekennzeichnet, dass** durch die Extraktion fluoreszierende Nebenprodukte aus dem Roh-N-Vinylamid bis auf einen Gehalt kleiner 0,05 Gew. Teile, bezogen auf 100 Gew. teile N-Vinylpyrrolidon entfernt werden.

## Claims

1. A process for removing by-products from N-vinylamide-rich product mixtures (crude N-vinylamide), which comprises performing an extraction of the crude N-vinylamide with an organic solvent as the extractant.

2. The process according to claim 1, wherein the N-vinylamide comprises cyclic N-vinylamides (vinyllactams) or noncyclic N-vinylamides of the formula 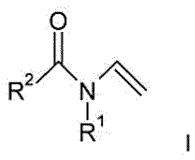 in which R1 and R2 are each independently a hydrogen atom or a C1 to C10 alkyl group.

3. The process according to claim 1 or 2, wherein the noncyclic N-vinylamides of the formula Comprise N-vinylformamide (R1 and R2 = H) or N-vinyl-N-methylacetamide (VIMA, R1 and R2 = methyl).

4. The process according to either of claims 1 and 2, wherein the cyclic N-vinylamides are N-vinylpiperidone, N-vinylcaprolactam or N-vinylpyrrolidone or derivatives thereof, for example N-vinylmethylpyrrolidone, N-vinyldimethylpyrrolidone or N-vinylethylpyrrolidone.

5. The process according to any one of claims 1 to 4, wherein the N-vinylamide is N-vinylpyrrolidone.

6. The process according to any one of claims 1 to 5, wherein the crude N-vinylamide consists of the N-vinylamide to an extent of at least 50% by weight.

7. The process according to any one of claims 1 to 6, wherein the crude N-vinylamide was obtained by reacting starting materials with acetylene and optionally a subsequent workup.

8. The process according to any one of claims 1 to 7, wherein the crude N-vinylamide is crude N-vinylpyrrolidone which was obtained by reacting 2-pyrrolidone (gamma-butyrolactam) with acetylene and optionally a subsequent workup.

9. The process according to any one of claims 1 to 8, wherein the extraction is preceded by performance of a distillation to remove the high boilers from the crude N-vinylamide (crude distillation).

10. The process according to any one of claims 1 to 9, wherein an aliphatic or cycloaliphatic solvent is used.

11. The process according to any one of claims 1 to 10, wherein the extraction is effective with addition of water.

12. The process according to any one of claims 1 to 11, wherein crude N-vinylpyrrolidone is extracted with methylcyclohexane with addition of water.

13. The process according to any one of claims 1 to 12, wherein the extraction is performed within a temperature range from 20 to 100°C.

14. The process according to any one of claims 1 to 13, wherein the extraction is performed continuously in one or more columns.

15. The process according to any one of claims 1 to 14, wherein crude N-vinylpyrrolidone is extracted continuously with methylcyclohexane with addition of water in a column, water being supplied via a distributor in the upper part of the methylcyclohexane via a distributor in the lower part of the column and the crude N-vinylpyrrolidone in the middle part of the column.

16. The process according to claim 15, wherein a mixture of methylcyclohexane and removed by-products is withdrawn at the top of the column, and a mixture of purified crude N-vinylpyrrolidone and water at the bottom of the column.

17. The process according to any one of claims 1 to 16, wherein the extraction removes fluorescent by-products from the crude N-vinylamide down to a content of less than 0.05 part by weight, based on 100 parts by weight of N-vinylpyrrolidone.

## Revendications

1. Procédé pour la séparation de produits secondaire d'avec des mélanges produits riches en N-vinylamide (N-vinylamide brut), **caractérisé en ce qu'**on effectue une extraction du N-vinylamide brut à l'aide d'un solvant organique en tant qu'agent d'extraction.

2. Procédé selon la revendication 1, **caractérisé en ce que** le N-vinylamide consiste en des N-vinylamides cycliques (vinyllactames) ou en des N-vinylamides non cycliques de formule 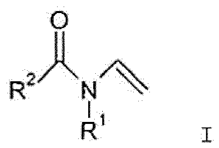 dans laquelle R¹ et R² représentent indépendamment l'un de l'autre un atome d'hydrogène ou un groupe alkyle en C₁-C₁₀.

3. Procédé selon la revendication 1 ou 2, **caractérisé en ce que** les N-vinylamides non cycliques de formule I consistent en N-vinylformamide (R¹ et R² = H) ou N-vinyl-N-méthylacétamide (VIMA, R¹ et R² = méthyle).

4. Procédé selon la revendication 1 ou 2, **caractérisé en ce que** les N-vinylamides cycliques consistent en N-vinylpipéridone, N-vinylcaprolactame ou N-vinylpyrrolidone ou ses dérivés, par exemple N-vinylméthylpyrrolidone, N-vinyldiméthylpyrrolidone ou N-vinyléthylpyrrolidone.

5. Procédé selon l'une quelconque des revendications 1 à 4, **caractérisé en ce que** le N-vinylamide consiste en N-vinylpyrrolidone.

6. Procédé selon l'une quelconque des revendications 1 à 5, **caractérisé en ce que** le N-vinylamide brut consiste à raison d'au moins 50 % en poids en le N-vinylamide.

7. Procédé selon l'une quelconque des revendications 1 à 6, **caractérisé en ce que** le N-vinylamide brut a été obtenu par mise en réaction de produits de départ avec de l'acétylène et éventuellement un traitement final subséquent.

8. Procédé selon l'une quelconque des revendications 1 à 7, **caractérisé en ce que** le N-vinylamide brut consiste en N-vinylpyrrolidone brute qui a été obtenue par mise en réaction de 2-pyrrolidone (gamma-butyrolactame) avec de l'acétylène et éventuellement un traitement final subséquent.

9. Procédé selon l'une quelconque des revendications 1 à 8, **caractérisé en ce qu'**avant l'extraction on effectue une distillation afin de séparer les composants à haut point d'ébullition du N-vinylamide brut (distillation de brut).

10. Procédé selon l'une quelconque des revendications 1 à 9, **caractérisé en ce qu'**on utilise un solvant aliphatique ou cycloaliphatique.

11. Procédé selon l'une quelconque des revendications 1 à 10, **caractérisé en ce que** l'extraction s'effectue avec addition d'eau.

12. Procédé selon l'une quelconque des revendications 1 à 11, **caractérisé en ce qu'**on extrait de la N-vinylpyrrolidone brute à l'aide de méthylcyclohexane avec addition d'eau.

13. Procédé selon l'une quelconque des revendications 1 à 12, **caractérisé en ce qu'**on effectue l'extraction dans une plage de température de 20 à 100 °C.

14. Procédé selon l'une quelconque des revendications 1 à 13, **caractérisé en ce qu'**on effectue l'extraction en contenu dans une ou plusieurs colonnes.

15. Procédé selon l'une quelconque des revendications 1 à 14, **caractérisé en ce qu'**on extrait de la N-vinylpyrrolidone brute en contenu dams une colonne à l'aide de méthylcyclohexane avec addition d'eau, en envoyant de l'eau par un distributeur dans la partie supérieure de la colonne, du méthylcyclohexane par un distributeur dans la partie inférieure de la colonne et la N-vinylpyrrolidone brute dans la partie médiane de la colonne.

16. Procédé selon la revendication 15, **caractérisé en ce qu'**on évacue à la tête de la colonne un mélange de méthylcyclohexane et de produits secondaires séparés et au pied de la colonne un mélange de N-vinylpyrrolidone brute purifiée et d'eau.

17. Procédé selon l'une quelconque des revendications 1 à 16, **caractérisé en ce que** par l'extraction on élimine des produits secondaires fluorescents à partir du N-vinylamide brut jusqu'à une teneur inférieure à 0,05 partie en poids, par rapport à 100 parties en poids de N-vinylpyrrolidone.
